# EUROPEAN PATENT APPLICATION

(11) **EP 2 008 532 A1**
(43) Date of publication of application: **31.12.2008**
(21) Application number: 07739987.1
(22) Date of filing: 28.03.2007
(51) Int. Cl.: A23L 1/30, A23L 2/52, A61K 9/08, A61K 9/107, A61K 31/22, A61K 36/81, A61K 47/14, A61K 47/36, A61K 47/38, A61P 3/00, A61P 37/04

(54) **METHOD FOR PRODUCTION OF CAPSINOID-CONTAINING BEVERAGE/FOOD**

(30) Priority: 31.03.2006 JP 2006098623
(71) Applicant: Ajinomoto Co., Inc., Tokyo 104-8315 (JP)
(72) Inventor: ITO, Yoko, Kawasaki-shi, Kanagawa 210-8681 (JP); WATANABE, Fumiko, Kawasaki-shi, Kanagawa 210-8681 (JP); MARUYAMA, Kentaro, Kawasaki-shi, Kanagawa 210-8681 (JP)
(74) Representative: Fischer, Heinrich
(86) International application number: PCT/JP2007/056549
(87) International publication number: WO 2007/114128

(57) **Abstract**

Provided is a method for producing a capsinoid-containing food/drink article with a superior stability of the capsinoid compound, and an emulsion composition comprising a capsinoid compound with an improved dilution stability thereof. The method comprises the steps of blending an oil phase containing a capsinoid compound with an aqueous phase and an emulsifier to prepare an emulsion composition, and mixing the emulsion composition with an aqueous diluent component, wherein an additional emulsifier and/or a high-molecular component is added to the emulsion composition, the aqueous diluent component or a mixture thereof.

## Description

### TECHNICAL FIELD

The present invention relates to a method for producing a capsinoid-containing food/drink article, and more particularly, to a method for producing a water-containing food/drink article by preparing an emulsion composition containing capsinoids and diluting the emulsion composition.

### BACKGROUND ART

It has been reported that a non-pungent cultivar of Capsicum, named "CH-19 sweet" which was selected and fixed by Yazawa et al. as a low hot pepper contains considerable amounts of new non-pungent capsinoids (see, for example, Non-Patent Document 1). The compounds belonging to capsinoids (fatty acid esters of vanillyl alcohol, capsiate, dihydrocapsiate, etc., hereinafter sometimes merely referred to as "capsinoid compound", "capsinoid" or "capsinoids") are non-pungent, different from pungent components of red pepper, capsaicinoids (capsaicin, dihydrocapsaicin, etc.), but they have been reported to exhibit an immune activating action, activation effect for energy metabolism, and the like (see Patent Document 1), and are expected to be put into practice in the future.

Capsinoids are very unstable because the ester linkage formed between a vanilloid group and a fatty acid chain is readily hydrolyzed. Therefore, a technique for solubilizing capsinoids in water with keeping stability has been required in order to add capsinoids into a liquid food product such as beverage (drink). As for such a technique, a technique for emulsifying an oil containing capsinoid with an emulsifier to yield an emulsified composition and solubilizing the latter in water, has been reported (see, for example, Patent Document 2).

[Patent Document 1] JP Patent Kokai Publication No. JP-A-11-246478
[Patent Document 2] JP Patent Kokai Publication No. JP-P2003-192576A
[Non-Patent Document 1] Yazawa et al., Engei Gakkai Zasshi (Journal of the Japanese Society for Horticultural Science) vol.58, 601-607 (1989)

### DISCLOSURE OF THE INVENTION

### Problems to be solved by the Invention

When an emulsion composition prepared according to a conventional way is merely diluted with an aqueous component to prepare a water-containing food/drink product such as aqueous beverage, there is a problem that the capsinoid compounds contained in the aqueous beverage are slowly decomposed during storage compared with a product immediately after production. In preparing a healthy functional food product, in order to assure a necessary amount exhibiting a physiological function through the term from the production up to the end of shelf life, the amount of the components to be incorporated (added) at the time of production is decided (increased) by estimating the amount decomposed during the shelf life. In preparing a healthy food product, it is desirable to reduce an extra amount to be added in view of the cost of manufacturing. Thus, it is desired that a method for increasing stability of capsinoid compounds in an aqueous solution is provided. In preparing a capsinoid-containing emulsion composition, increase of the concentration of an emulsifier may slightly improve the stability upon dilution, but there is a possibility that the presence of a large quantity of emulsifier during emulsification may have a bad influence on an emulsifying action. Thus, there is a desire for a method for stably producing a capsinoid-containing aqueous food/drink article and a capsinoid-containing aqueous emulsion composition used therein of which the dilution stability is improved.

### Means for Solving the Problems

The present inventors worked assiduously to solve the problems as mentioned above and as a result they found that addition of an additional emulsifier and/or a high-molecular component upon dilution of a capsinoid-containing emulsion composition with an aqueous component raises the stability of capsinoid when an oil phase containing the capsinoid compound is incorporated into an aqueous phase in an emulsified state.

That is, a method for producing a capsinoid-containing food/drink article according to the present invention comprises blending an oil phase containing a capsinoid compound with an aqueous phase and an emulsifier to prepare an emulsion composition; and mixing the emulsion composition with an aqueous diluent component (may be termed "aqueous component" hereinafter), wherein an additional emulsifier and/or a high-molecular component is added to the above-mentioned emulsion composition, aqueous diluent component or a mixture thereof. The additional emulsifier and/or high-molecular component may be added in the step in which the above-mentioned emulsion composition is mixed with the aqueous diluent component, or may be added to any one or both of the above-mentioned emulsion composition and the above-mentioned aqueous diluent component before mixing the above-mentioned emulsion composition with the aqueous diluent component. In addition, the stability of the capsinoid-containing emulsion composition can be increased by further adding an oil thickener to the above-mentioned oil phase.

In another aspect of the invention, the invention provides an emulsion composition with an improved dilution stability of a capsinoid compound wherein an emulsion composition prepared by blending an oil phase containing a capsinoid compound, an aqueous phase and an emulsifier, is further admixed by an additional emulsifier and/or a high molecular component.

### Effect of the invention

According to the present invention, the capsinoid compound contained in the food/drink article prepared by diluting a capsinoid-containing emulsion composition using the aqueous component is improved in its stability, and thus, the improvement is considered to allow an extension of the shelf life of the product and contribute to reduction of the cost of manufacturing.

### BRIEF DESCRIPTION OF THE DRAWINGS

Fig. 1 shows a storage stability of dihydrocapsiate in a medium containing decaglycerin monooleate (trade name: Decaglyn 1-0).
Fig. 2 shows a storage stability of dihydrocapsiate in media containing 8 sorts of emulsifiers.
Fig. 3 shows a storage stability of dihydrocapsiate in media containing 8 sorts of high-molecular components.
Fig. 4 shows storage stability of dihydrocapsiate when a capsinoid-containing emulsion preparation was diluted in different ways. (A) indicates a case where a capsinoid-containing emulsion preparation was diluted with an aqueous buffer containing an emulsifier; (B) indicates a case where a capsinoid-containing emulsion preparation, to which was added an emulsifier in advance, was diluted with an aqueous buffer containing no emulsifier.

### PREFERRD MODES FOR CARRYING OUT THE INVENTION

In the following, the invention will be explained in details with reference to preferred modes, which are not intended to limit the scope of the invention.

### (Capsinoid compounds)

In this invention, capsinoid compounds mean fatty acid esters of vanillyl alcohol; the representative components thereof encompass those confirmed as components contained in capsicum, comprising capsiate, dihydrocapsiate, and nordihydrocapsiate; further comprising fatty acid esters including vanillyl alcohol and a variety of straight or branched chain fatty acids which have approximately the same fatty acid length as capsiate or nordihydrocapsiate such as vanillyl decanoate, vanillyl nonanoate, vanillyl octanoate, and the like. Capsiate (hereinafter sometimes abbreviated to "CST"), dihydrocapsiate (hereinafter sometimes abbreviated to "DCT") and nordihydrocapsiate (hereinafter sometimes abbreviated to "NDCT") can be represented by the following chemical formulae.

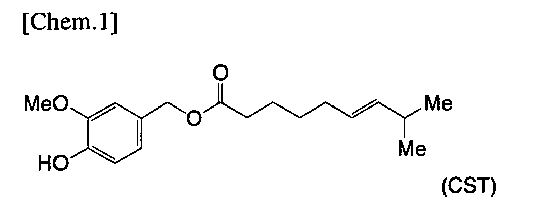

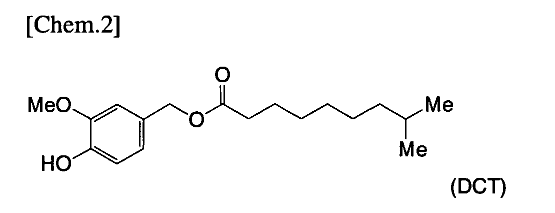

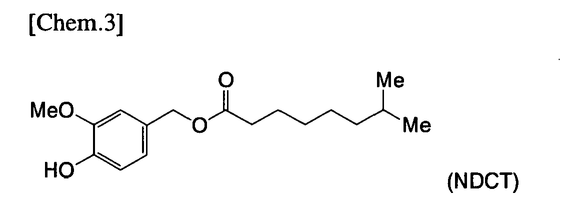

Capsinoids are abundant in the plant body belonging to a genus Capsicum (hereinafter referred to "red pepper") and can be prepared by purification and separation from the plant body and/or fruit of red pepper. There is no limitation in species of red pepper used in purification as far as it contains capsinoids, and it is preferable to use a non-pungent variety of red pepper, though one derived from a native variety of pungent red pepper exemplified by "Nikko" and "Goshiki" may be used. In particular, non-pungent varieties such as "CH-19 sweet", "Man-ganji", "Fushimi Kancho", and the like, or green pepper, sweet green pepper, and the like can preferably be employed since they contain much capsinoid compounds. A non-pungent variety "CH-19 sweet" is particularly preferred because the content of said components is particularly high. The term "CH-19 sweet" herein means a variety "CH-19 sweet" and its descendant relatives derived from"CH-19 sweet". Purification and separation of the capsinoid compounds can be carried out in a conventional way well known by a person skilled in the art, for example, extraction with a solvent, a variety of chromatography such as silica gel chromatography, preparative high performance liquid chromatography, etc. alone or in combination thereof, as fit. For example, the method as described in the above-mentioned Patent Document 1 may be used.

Alternatively, the above-mentioned capsinoid compounds may be synthesized by an ester exchange reaction of the corresponding fatty acid ester and vanillyl alcohol as starting materials, for example, as described in the above-mentioned Patent Document 1. It is also possible to synthesize based on its chemical structure according to other reaction techniques well known by a person skilled in the art. Moreover, capsinoids can readily be prepared by a synthetic process using an enzyme. For example, a desired capsinoid compound can readily be obtained by a reverse reaction of lipase using a fatty acid ester corresponding to the desired compound, and/or a compound such as triglyceride containing the fatty acid concerned, and vanillyl alcohol as substrates, according to the method as described in JP-A-2000-312598 or Kobata et al., Biosci. Biotechnol. Biochem., 66(2), 319-327 (2002).

The capsinoid compounds, when used in the method of production in the invention, may be any of the above-mentioned extraction products or synthetic products, and they may be a single compound or a mixture of two or more compounds. Further, the capsinoid compounds to be used may contain their decomposition products, i.e., free fatty acids or vanillyl alcohol etc.

### (Oil phase)

The oil phase containing a capsinoid compound means a fat-soluble material prepared by blending the above-mentioned capsinoid compound with fats and oils. These fats and oils include, for example, vegetable oils such as soybean oil, coconut oil, rice oil, corn oil, palm oil, safflower oil, rapeseed oil, olive oil, and the like; middle chain saturated fatty acid triglycerides (hereinafter also referred to as "MCT") constituted by glycerin and fatty acids including saturated fatty acids of 6 to 10 carbon atoms (for example, capric acid, caprylic acid, etc.) as major constituents; animal fats and oils such as beef tallow, lard, chicken fat, and fish oil; fatty acids such as oleic acid; and mixtures thereof. In addition, sucrose acetate isobutyrate (SAIB), various Rosin esters, and Elemi resins may be added as a specific gravity adjuster.

### (Oil thickener)

The oil phase containing a capsinoid compound preferably contains the following oil thickener in addition to the above-mentioned fats and oils. There is no limitation in an oil thickener used in the invention as far as it shows a thickening effect when dissolved in an oil preparation, including for example fat and oil-solidifying agent such as long chain fatty acid esters. As the long chain fatty acid esters, those of 20 or more carbon atoms are preferred. The fatty acid esters of 20 or more carbon atoms, for example, include esters of arachic acid, behenic acid, etc. with propylene glycol, glycerin, sorbitan, pentaerythritol, diglycerin, etc. Specifically, the esters include, but are not limited to, glycerin monobeheniate, glycerin monoarachiate, sorbitan diarachiate, and the like. In particular, hexaglycerin octastearate, monoglycerin monobeheniate, and their mixture are preferred, and specifically a commercially available "TAISET" (trade name by Taiyo Chemical Co.) is exemplified.

The amount of the oil thickener to be added is determined as an amount of the thickener by which the viscosity of hydrophobic component before addition of the thickener is improved, for example the amount being in 0.1 - 10% by mass, preferably 0.5 - 5% by mass, more preferably 1 - 3 % by mass for the total of the oil phase. Thus, the viscosity of the capsinoid-containing oil phase is adjusted preferably to a viscosity of 50 - 350 mPa·s by addition of the oil thickener. The viscosity can be determined by a conventional method well known by a person skilled in the art, for example, using a conventional measuring apparatus such as rotary viscosimeter, capillary viscosimeter, falling sphere viscosimeter, and the like.

### (Aqueous phase)

The aqueous phase is used in the invention in preparation of an oil-in-water type emulsion composition based on emulsifying the above-mentioned oil phase. If required, saccharides such as sugar, glutinous starch syrup, etc., polyhydric alcohols such as glycerin, sorbitol, propylene glycol, etc., organic acids such as citric acid, malic acid, etc., salts such as sodium chloride, potassium chloride, calcium chloride, etc., anti-oxidant, □-carotene, pigments such as paprika pigment, etc. may properly be blended. The aqueous phase may preferably be adjusted to an acidic range, more preferably pH 2 - 6. The acidic material used in adjusting the pH includes, but not particularly limited to, for example, organic acids or their salts such as citric acid, adipic acid, succinic acid, tartaric acid, lactic acid, fumaric acid, DL-malic acid, benzoic acid, gluconic acid, gluconodeltalactone, and the like; salts such as potassium carbonate, sodium bicarbonate, sodium carbonate, sodium dihydrogen pyrophosphate, and the like; inorganic acids and their salts such as phosphoric acid; vitamin C and its salts. These acidic materials are used in adjustment of the pH of an emulsion composition to an acidic range, allowing maintenance of the stability of capsinoid compounds for a long term storage.

### (Emulsifier)

There is no particular limitation in emulsifiers used in preparation of a capsinoid-containing emulsion composition, and a variety of emulsifiers so far used in food and drink can be used. For example, monoglycerin fatty acid esters, diglycerin fatty acid esters, triglycerin fatty acid esters, monoglycerin fatty acid ester derivatives, propylene glycol fatty acid esters, sucrose fatty acid esters, polyglycerin fatty acid esters, polyoxyethylene sorbitan fatty acid esters, lecithin, modified starch, sorbitan fatty acid esters, gum arabic, gum ghatti, pectin, carrageenan, octinylsuccinic acid starch and quillai extract are included.

In particular, polyglycerin fatty acid esters are preferred in view of stability of emulsification. The amount of these emulsifiers to be used is not strictly limited but they may be properly used in the wide range of amount depending on their sort. Usually, the emulsifier may be used in an amount of approximately 2 - 30% by mass, preferably approximately 3 - 25% by mass, for the total of an oil phase, aqueous phase and emulsifier. For example, when a polyglycerin fatty acid ester is used as an emulsifier, it is preferably used within the range of approximately 6 - 10% by mass. In such a case, the ratio of an emulsifier, an oil phase and an aqueous phase to be blended may be determined so that an emulsion composition of oil-in-water type is formed; a person skilled in the art can easily determine it according to the sort of oil and emulsifier chosen.

In producing a capsinoid-containing emulsion composition, an oil phase containing a capsinoid compound, preferably an oil phase containing the above-mentioned oil thickener, is blended with an aqueous phase and an emulsifier, and properly emulsified by a conventional emulsifying method for fats and oils to prepare an emulsion composition. In a preferred embodiment, for example, an oil phase containing a capsinoid compound is first blended with an aqueous phase containing an emulsifier (the above-mentioned emulsifier is dissolved in water with heating), and if required adjusting the resulting emulsion composition at pH 2 - 6 with the above-mentioned acidic material, followed by mixing with a homomixer, colloid mill, high pressure homogenizer, and the like, to prepare an emulsion composition in which the capsinoid compound is highly stable.

After preparation of the above-mentioned emulsion composition, an additional emulsifier and/or a high molecular component may further be added. Increase of the concentration of an emulsifier and/or a high molecular component in an emulsion composition is effective in improvement of the stability of the capsinoid compound after dilution as described below.

### (Method for producing a capsinoid-containing food/drink article in the present invention)

The capsinoid-containing food/drink article of the invention (hereinafter referred to as "food and drink product of the invention") is prepared by mixing the above-mentioned emulsion composition with an aqueous diluent component, that is, diluting the emulsion component with an aqueous component, wherein the invention is featured by that an additional emulsifier and/or a high molecular component is further added to the above-mentioned emulsion composition, aqueous component or their mixture at any step of producing the above-mentioned food and drink product. In this connection, the aqueous diluent component is a component constituting food and drink article other than the emulsion component to be added, including for example an aqueous solution, oil-in-water type emulsion, gell-like solution, and the like. In this connection, the aqueous solution is basically the same as the aqueous phase used in preparation of an emulsion composition. In a case of preparing an aqueous drink product as a food and drink product, an aqueous solution containing fruit juice, vitamins, amino acids, perfume, sugars, and the like is preferably added to a capsinoid-containing emulsion composition to form a refreshing drink or a carbonated drink. In a case of using an oil-in-water type emulsion as an aqueous diluent component, frozen dessert such as ice-cream, a variety of dairy products such as yoghurt or milk, a variety of emulsified food and drink products such as custard pudding, mousse, bavarois, dressing, and the like can be produced. In a case of using a gell-like solution as an aqueous diluent component, a gell-like food product such as jelly can be produced. These food and drink products preferably contain at least 10% by mass of moisture. The capsinoid-containing emulsion composition blended into these food and drink products may be used in the range of the amount by which oil drops contained in the above-mentioned emulsion composition are maintained, generally in the range of about 0.01 - about 5% by mass for the food and drink product, though it changes depending on the purpose of use, sort of food and drink product, and form.

In a preferred embodiment of the invention, an additional emulsifier and/or a high molecular component is added at the step in which the above-mentioned emulsion composition is mixed with an aqueous diluent component. Alternatively, it may be added to a capsinoid-containing emulsion composition and/or an aqueous diluent component in advance. The above-mentioned emulsifier may be the same as that used in preparation of the emulsion composition, and in particular, polyglycerin fatty acid ester, sorbitan fatty acid ester, sucrose fatty acid ester, organic acid glycerin fatty acid ester, and polyoxyethylene sorbitan fatty acid ester are preferably used. These emulsifiers may be used alone or in combination of two or more species. The amount to be added may properly be optimized depending on the sort or utility of the food and drink product to be produced, with no particular limitation, and it is, for example, 0.01 - 5 % by mass, preferably 0.05 - 1% by mass for the total of the food and drink product.

On the other hand, the high molecular component includes naturally occurring or synthetic polysaccharides or proteins. The polysaccharides include gum arabic, gum ghatti, tragacanth gum, guar gum, karaya gum, xanthan gum, pectin, alginic acid and its salts, carrageenan, cellulose and its derivatives. The proteins include soybean protein, gelatin, collagen, casein, casein sodium, and the like, and these may be used alone or in combination of two or more. In particular, carrageenan, HM pectin (high methoxyl pectin), and carboxymethylcellulose sodium (CMC sodium) are preferred. Carrageenan is a naturally occurring high molecular substance of a molecular weight being 100,000 - 500,000, extracted and purified from a sea weed, red algae, mainly composed of galactose and 3,6-anhydrogalactose. Pectin is a naturally occurring high molecular polysaccharide mainly composed of galacturonic acid and methylgalacturonic acid; pectin in which methylgalacturonic acid occupies 50% or more to the total galacturonic acid is called HM pectin, while one containing lower than 50% called LM pectin. HM pectin having 55% or more of sugar content is readily gelled around pH 3. CMC sodium is also called sodium cellulose glycolate and has been used as a thickener in food additives. The amount to be added may properly be optimized depending of the sort or utility of aqueous drink products with no particular limitation, and for example, it may be in 0.01 - 5% by mass, preferably 0.1 - 1% by mass for the total of the aqueous drink product.

The products produced by the method of the invention may be accompanied by a labeling indicating that these water-containing food and drink products activate an immune system, activate energy metabolism, and can be used in improvement of lifestyle related diseases. The labeling may be indicated on a package of the product or incorporated into a package as a package insert. In such indication, it is preferable to involve a specification describing a proper does per day and the manner of administration.

The invention will be further explained by way of specific examples, which are not intended to limit the invention. In this connection, the symbol % used in Examples means % by mass.

### EXAMPLES

### [Example 1] Preparation and evaluation of stability of a diluted solution of a capsinoid emulsified preparation added with an emulsifier:

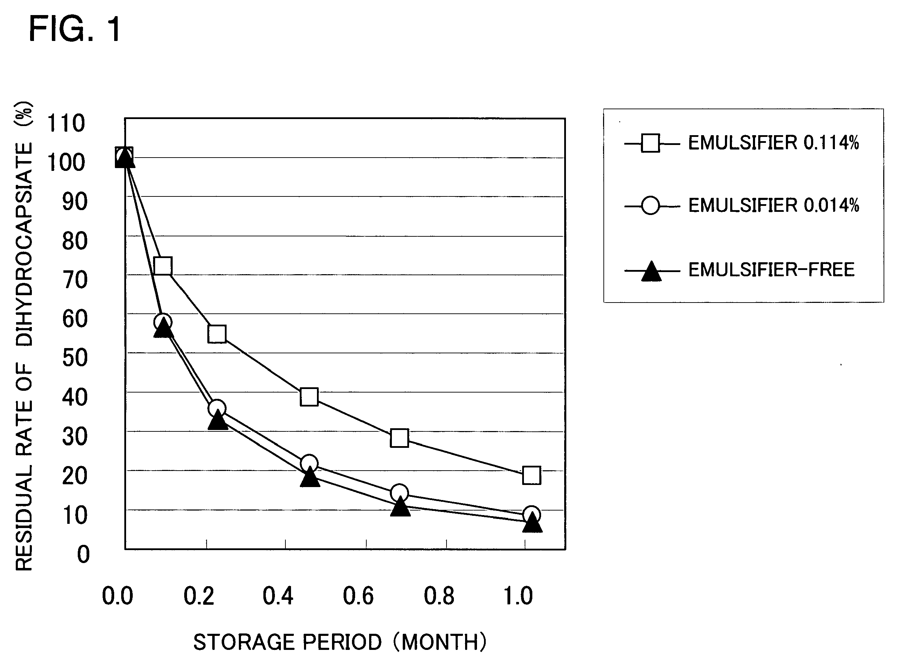

According to a method as described in Example 1 of the above-cited Patent Document 2, a dihydrocapsiate-containing emulsion composition was prepared. Briefly, decaglycerin monooleate (Nikko Chemical Co.; trade name, Decaglynl-O)(21 g), glycerin (193 g) and water (26 g) were mixed, dissolved under heating at 90-95°C, and cooled to 30°C. To the resultant mass was added synthetic dihydrocapsiate (8.5 g) and sucrose acetate isobutyrate (SAIB)(51 g) dissolved under heating at 85 - 90°C, and the mixture was emulsified at 30 - 90°C to prepare a capsinoid-containing emulsion composition (pH 3.6). The resulting emulsion composition was diluted into 1000 times with 0.01M citrate buffer (pH 3.5) supplemented with sucrose to obtain an additive-free sample. On the other hand, the composition was diluted into 1000 times with 0.01M citrate buffer (pH 3.5) supplemented with sucrose to which was added 0.014%, 0.114% emulsifier (Nikko Chemical Co.; Decaglyn 1-0) respectively, to prepare emulsion-added (supplemented) samples. The resulting samples were sterilized under heating for 20 minutes and stored at 44°C. Every one week or so after storage, the capsinoid content was determined. Analysis of the capsinoid content was performed by high performance liquid chromatography (HPLC) after dilution with an ethyl acetate/methanol (6 : 4) mixture solvent. Fig. 1 shows the result. As shown in Fig. 1, the result indicates that the storage stability of dihydrocapsinoate is increased with increase of the amount of emulsifier added. In a sample to which 0.114% emulsifier was added, the rate of residual dihydrocapsiate was 2 times and above more stable than the emulsifier-free case about one month after storage.

### [Example 2] Preparation and evaluation of stability of a diluted solution of a capsinoid emulsified preparation to which a variety of emulsifiers are added:

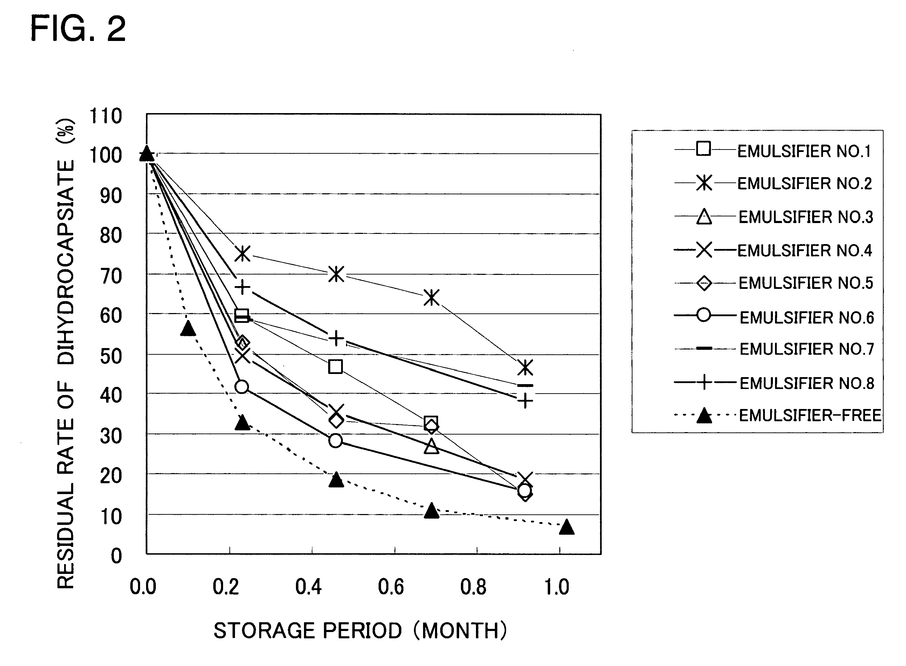

In the same manner as in Example 1, a dihydrocapsiate-containing emulsion composition was diluted into 1000 times with 0.01M citrate buffer (pH 3.5) supplemented with sucrose and the stability of storage was evaluated. To 0.01M citrate buffer (pH 3.5) supplemented with sucrose were added, respectively, 8 sorts of emulsifiers so as to be 0.114% in advance, and differences in the stability depending on the emulsifiers added were evaluated. Table 1 indicates 8 species of emulsifiers added, and the results of storage are shown in Fig. 2. From the results of Fig. 2, it was recognized that even emulsifiers other than Decaglyn 1-0 have the effect for improving the stability of dihydrocapsiate during storage.

**[Table 1]**

| Emuls. No. | Sorts of Emulsifiers | Trade Name | Manufacturer |
|---|---|---|---|
| 1 | Polyoxyethylene (20) sorbitan monooleate | Tween 80 | Sigma-Aldrich |
| 2 | Polyoxyethylene (5) sorbitan monooleate | Tween 81 | Sigma-Aldrich |
| 3 | Polyoxyethylene (20) sorbitan monolaurate | Tween 20 | Sigma-Aldrich |
| 4 | Decaglycerin monomyristate | - | Taiyo Chem. Co. |
| 5 | Decaglycerin pentaoleate | - | Taiyo Chem. Co. |
| 6 | Citric acid monoglycerin stearate | SunSoft621B | Taiyo Chem. Co. |
| 7 | Sucrose stearate | S1670 | Mitsubishi Chem. Co. |
| 8 | Sorbitan monooleate | Span 80 | Sigma-Aldrich |

### [Example 3] Preparation and evaluation of stability of a diluted solution of a capsinoid emulsified preparation to which a variety of high-molecular components are added:

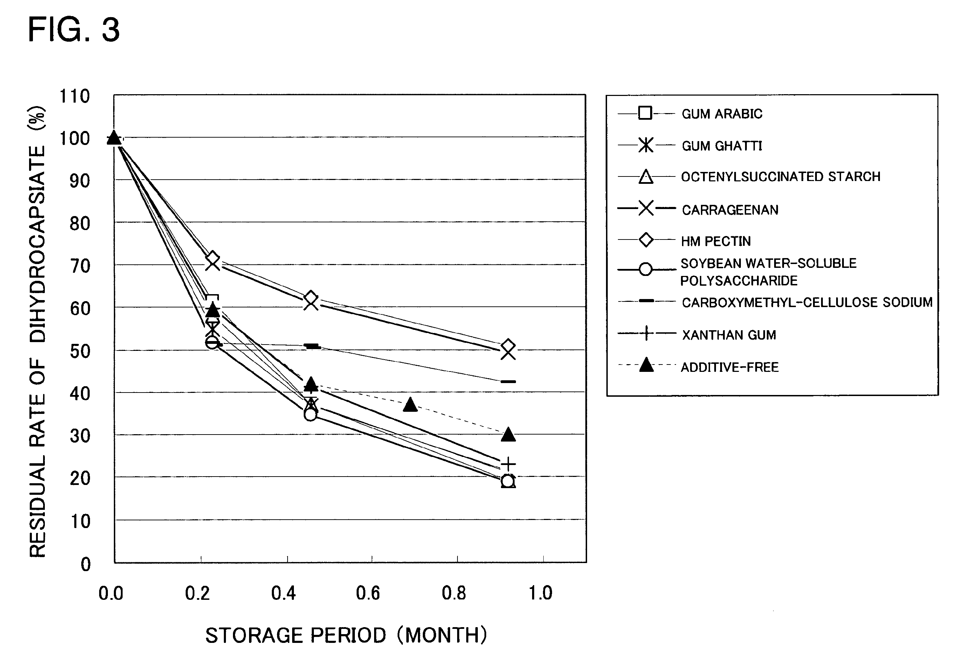

In the same manner as in Example 2, a dihydrocapsiate-containing emulsion composition was diluted into 500 times with 0.01M citrate buffer (pH 3.5) supplemented with sucrose and the stability of storage was evaluated. To 0.01M citrate buffer (pH 3.5) supplemented with sucrose were added, respectively, 8 sorts of high-molecular components in advance, and differences in the stability depending on the high-molecular components added were evaluated. Table 2 shows the high-molecular component samples evaluated. Regarding the amount of high-molecular component to be added, xanthan gum was reduced to 0.1% because it gives high viscosity in a small quantity. The amount of high-molecular components added other than xanthan gum was fixed at 0.5%. The results of storage are shown in Fig. 3. Improvement in the storage stability of dihydrocapsiate was recognized in HM pectin (0), carrageenan (×), and carboxymethylcellulose sodium (CMC sodium)(-).

**[Table 2]**

| Hi Mol. Comp. No. | Sort | Trade Name | Manufacturer |
|---|---|---|---|
| 1 | Gum arabic | Super gum EM2 | Saneigen FFI |
| 2 | Gum ghatti | Gum Ghatti SD | Saneigen FFI |
| 3 | Octenylsuccinated starch | Emulstar#30 | Matsutani Chem. Ind. |
| 4 | Carrageenan | Carrageenin CSL-1 | Saneigen FFI |
| 5 | HM pectin | YM-150-LJ | CP KELCO |
| 6 | Soybean water-soluble polysaccharide | Soybean Food fiber SM-1200 | Saneigen FFI |
| 7 | Carboxymethyl-cellulose sodium | FTS-1 | Nippon Syn. Chem. Ind. |
| 8 | Xanthan gum | Sun Ace NXG-S | Saneigen FFI |

### [Example 4] Preparation of a composition in which a capsinoid emulsion composition was mixed with an emulsifier in advance, and evaluation of the stability in a diluted solution:

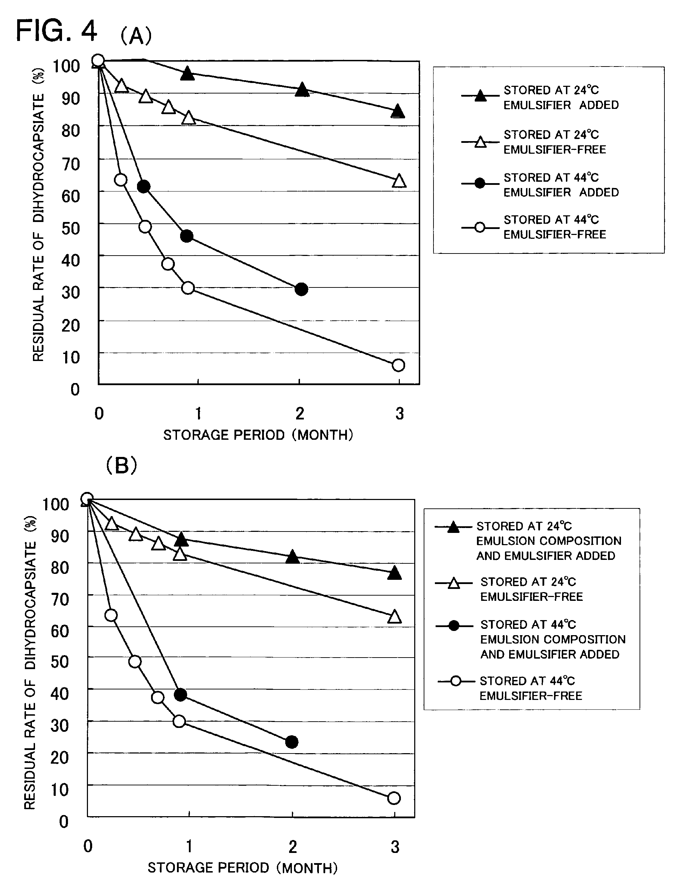

Into a capsinoid emulsion composition prepared in Example 1 was blended an emulsifier (Nikko Chemical Co., Decaglyn 1-0) at a ratio of 10 to 3 under heating (50 - 70°C) to prepare a composition. The resulting composition was diluted into 230 times with 0.01M citrate buffer (pH 3.5) supplemented with sucrose to prepare a mixture sample of the emulsion composition and the emulsifier. On the other hand, an additive-free sample was prepared by diluting the capsinoid emulsion composition (prepared in Example 1) into 300 times with the same buffer; and a sample containing an emulsifier was prepared by diluting the capsinoid emulsion composition (prepared in Example 1) into 300 times with 0.01M citrate buffer (pH 3.5) supplemented with sucrose to which was added an emulsifier (Nikko Chemical Co., Decaglyn 1-0) in advance at a rate of 0.10%. In either sample, the concentration of capsinoid after dilution with the buffer correspond to 0.33 % of the initial amount of capsinoid existing in the emulsion composition (preparation). Thus prepared samples were sterilized under heating for 20 minutes and stored at 24°C and 44°C. In periodical sampling, the content of capsinoid was determined. The content of capsinoid was analyzed by high performance liquid chromatography (HPLC) after dilution with a mixture of ethyl acetate and methanol (6 : 4). Fig. 4 shows the results. Fig. 4A indicates, similarly in Example 1, that dilution of the emulsion composition with a buffer to which was added an emulsifier in advance improves the storage stability of dihydrocapsiate. Further, Fig. 4B indicates that dilution of the emulsion composition, to which an emulsifier was added in advance, with a buffer containing no emulsifier, also improves the storage stability of dihydrocapsiate, similarly in case of A.

### INDUSTRIAL APPLICABILITY

The method of the invention is utilizable in the fields of food products and cosmetic or the like.

## Claims

1. A method for producing a capsinoid-containing food/drink article, comprising
preparing an emulsion composition by blending an oil phase containing (at least) a capsinoid compound with an aqueous phase and (at least) an emulsifier ; and
mixing said emulsion composition with (at least) an aqueous diluent component,
**characterized in that** (at least) an additional emulsifier and/or (at least) a high-molecular component is added to said emulsion composition, said aqueous diluent component or a mixture thereof.

2. The method of claim 1, **characterized in that** said additional emulsifier and/or said high-molecular component is added in the step of mixing the emulsion composition with the aqueous diluent component.

3. The method of claim 1, **characterized in that** said additional emulsifier and/or said high-molecular component is added to one or both of the emulsion composition and the aqueous diluent component, in advance of said mixing the emulsion composition with the aqueous diluent component.

4. The method of any one of claims 1 to 3, **characterized in that** said oil phase further comprises an oil thickner.

5. The method of any one of claims 1 to 4, **characterized in that** said emulsifier is polyglycerol fatty acid ester, sorbitan fatty acid ester, sucrose fatty acid ester, plyoxyethylene sorbitan fatty acid ester, and/or organic acid glycerate fatty acid ester.

6. The method of any one of claims 1 to 5, **characterized in that** said high-molecular component is carrageenan, HM pectin, and/or sodium carboxymethylcellulose.

7. The method of any one of claims 1 to 6, **characterized in that** said food/drink article has a water content of at least 10% by mass.

8. The method of any one of claims 1 to 7, **characterized in that** said food/drink article is an aqueous drink, a dairy product or an ice-cold confectionery.

9. A capsinoid-containing food/drink article **characterized by** being produced by the method of any one of claims 1 to 8.

10. An emulsion composition with an improved dilution stability of a capsinoid compound, **characterized in that** an emulsion composition prepared by blending an oil phase containing (at least) a capsinoid compound, an aqueous phase and (at least) an emulsifier is further admixed by (at least) an additional emulsifier and/or (at least) a high-molecular component.
